# EUROPEAN PATENT SPECIFICATION

(11) **EP 2 482 935 B1**
(45) Date of publication and mention of the grant of the patent: **13.04.2016**
(21) Application number: 10763860.3
(22) Date of filing: 26.09.2010
(51) Int. Cl.: A63B 71/06, A63B 24/00

(54) **SYSTEM FOR SUPPORTING A USER TO DO EXERCISES**
SYSTEM ZUR UNTERSTÜTZUNG DES TRAININGS EINES BENUTZERS
SYSTÈME DE SOUTIEN D'UN UTILISATEUR POUR L'EXÉCUTION D'EXERCICES

(30) Priority: 30.09.2009 CN 200910174124
(43) Date of publication of application: 08.08.2012
(73) Proprietor: Koninklijke Philips N.V., 5656 AE Eindhoven (NL)
(72) Inventor: SONG, Rong, Shanghai 200233 (CN); DING, Chao, Shanghai 200233 (CN); PENG, Yang, Shanghai 200233 (CN); ZHANG, Yuan, Shanghai 200233 (CN)
(74) Representative: van Velzen, Maaike Mathilde
(86) International application number: PCT/IB2010/054326
(87) International publication number: WO 2011/039689

(56) References cited:
- EP-A1- 2 072 094
- WO-A1-2006/034571
- WO-A1-2007/058512
- WO-A1-2009/029834
- US-A- 5 078 152
- US-A1- 2008 255 479

## Description

### Technical field

*1* The present invention relates to a system for supporting a user to do exercises; more specifically, it relates to a method and device for automatically controlling the system.

### Background of the invention

*2* Cerebrovascular diseases, such as stroke, brain trauma cause many dysfunctions, for example, mobility dysfunction, speech dysfunction and cognitive dysfunction. It is widely known that professional physiotherapy helps with the rehabiliatation and also makes the rehabilitation process more predictable. It is also widely known that physical exercise helps in body building. It is important for a person to get professional guidance and do tailored exercises so as to obtain a better rehabilitation effect.

*3* However, a good rehabilitation effect could not be often achieved since persons frequently do exercises incorrectly because of pain, misunderstanding the instructions given or simply forgetting some or all of the content associated with tailored exercises, etc. Due to the fact that physical therapy (PT) is quite labor intensive and time consuming and that there is a shortage of skilled PT therapists, not every patient can get sufficient therapy and guidance via one-to-one manual therapy.

Based on the above, a need exists to provide a system for supporting a user to do a set of exercises, and replace the current one-to-one manual therapy with a one-to-many therapy scenario, so that a therapist can periodically supervise and guide multiple patients simultaneously so as to increase therapy efficiency.

US5078152 discloses a method for diagnosis and/or training of proprioceptor feedback capabilities of a muscle and joint system of a human patient using an exercise system having a patient attachment device and an arrangement for controlling parameters of an exercise movement in response to a control signal derived from one or more measured exercise parameters.

WO2007/058512 discloses a gait trainer which provides exercise prescription system using the gait trainer. The gait trainer comprises a display, pedals on which user's feet are placed, a control unit which receives physical information about the physical condition of the user while the pedals are moving, defines an optimal exercise prescription based on the physical information, and provides the defined exercise prescription to the display.

WO2006/034571 discloses a system which allows training and qualification of a user performing a skill-related training exercise involving body motion in a workspace. A training environment is selected through computer apparatus, and variables, parameters and controls of the training environment and the training exercise are adjusted.

EP2072094 discloses a sound producing device which uses physiological information to create sound outputs based on the user's physiological data, without individual differences between human instructors who provide the instructions form human movement, by using sounds that regulate human movement by machine.

### Summary of the invention

*4* In order to overcome the drawback of the prior art, the present invention provides a system according to claim 6 for supporting a user to do exercises, as well as a device and a method according to claim 1 for controlling such a system.

*5* According to an embodiment of the present invention, a method of controlling a system to support a user to do exercises is proposed. The system comprises a first subsystem for providing audio/video content related to a set of exercises, said set of exercises being associated with said user. The method comprises a step of acquiring previously generated data related to the user. This user related data comprises at least one of information relating to said set of exercises and characteristics of said user. The method also comprises a step of adjusting a parameter of the first subsystem according to said data.

*6* According to another embodiment of the present invention, a device for controlling a system to support a user to do exercises is proposed. The system comprises a first subsystem for providing audio/video content related to a set of exercises, said set of exercises being associated with said user. The device comprises a first unit for acquiring previously generated data related to the user. This user related data comprises at least one of information relating to said set of exercises and characteristics of said user. The device also comprises a second unit for adjusting a parameter of the first subsystem according to said data.

According to another embodiment of the present invention, a system for supporting a user to do a set of exercises is proposed. The system comprises a first subsystem for providing audio/video content related to said set of exercises. The system also comprises the above-mentioned device for controlling said system.

The system further comprises a second subsystem for supporting the user's body when the user does said set of exercises.

Accordingly, the device for controlling such a system further comprises a third unit for adjusting a parameter of the second subsystem according to the previously generated data.

Accordingly, the method of controlling such a system comprises a step of acquiring the previously generated data, and at least one of the following steps: adjusting a parameter of the first subsystem according to said data; adjusting a parameter of the second subsystem according to said data. *7* By using the system, the user could receive necessary instructions and do tailored exercises so as to obtain a good exercising effect. By having such a controlling device in the system, the system can be adapted automatically to fit different users according to the condition of the individual users, without interacting with the user. This is very helpful when the user has difficulties adjusting the system by himself.

### Brief description of the drawings

*8* Other features, objects and advantages of the present invention will become more apparent from the following detailed description of non-limited exemplary embodiments, when taken in conjunction with the accompanying drawings:
Fig.1 illustrates a system according to an embodiment of the present invention;
Fig.2 illustrates a flowchart of a method according to an embodiment of the present invention;
Fig.3 illustrates a system according to another embodiment of the present invention;
Fig.4 illustrates a system according to another embodiment of the present invention;
Fig.5a and Fig.5b illustrate a system according to another embodiment of the present invention;
Fig.6 illustrates a system according to another embodiment of the present invention;
Fig.7 illustrates a flowchart of a method according to another embodiment of the present invention.
Same or similar reference signs refer to same or similar apparatuses or circuits.

### Detailed description of embodiments

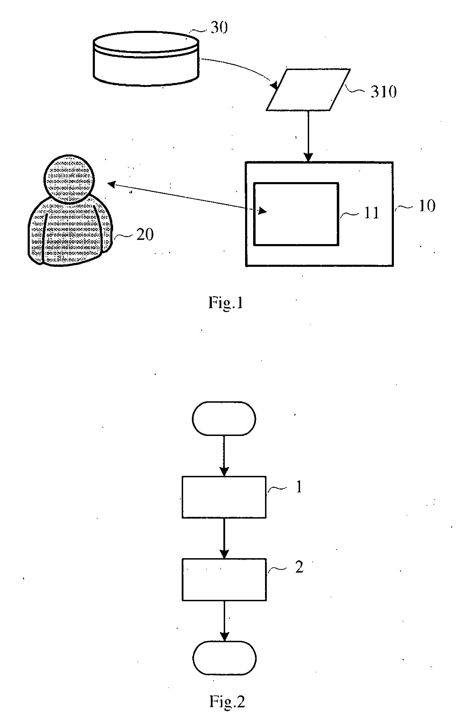

*9* Fig.1 illustrates the system according to an aspect of the present invention. As shown in Fig.1, there are a user 20 and a system 10.

*10* For example, the user 20 is a patient with a mobility dysfunction and needs rehabilitation training. The user 20 could for example follow a training plan generated by a doctor or a therapist. The training plan at least comprises a set of exercises intended to be done by the user 20. The set of exercises may refer to one exercise or multiple exercises.

*11* The system 10 is used for supporting the user 20 to do the exercises. The system 10 comprises a first subsystem 11 for providing audio/video content related to said set of exercises associated with the user 20. The user 20 could follow the audio/video content to do the exercises according to the training plan.

*12* Fig.2 illustrates a flowchart for controlling the system 10 to support the user 20 to do the exercises. As shown in Fig.2, the method comprises two steps. Step 1 serves for acquiring previously generated data 310 which is related to the user 20. Step 2 serves for adjusting a parameter of the subsystem 11 according to the data 310.

*13* It should be understood that, since the invention aims at automatic adjustment of the system without user interaction, in the context of this invention, the data 310 is generated prior to step 1. It means that the data 310 is not generated during the period of performing step 1. It is already available and stored somewhere inside or outside the system 10. For example, the data 310 could be medical records of the user 20, generated by a doctor or a therapist when the user 20 came to see the doctor or the therapist. In other words, the data 310 is a result of examination and/or diagnosis in a medical institution. The data 310 may also be generated by any other person and any other entity.

*14* The data 310 may comprise information relating to the above mentioned set of exercises. The data 310 may also comprise information relating to characteristics of the user 20, such as height, weight, symptom, medical record, and the like. The data 310 could either be occasionally acquired from outside the system 10, or be pre-stored in the system 10. For example, it may be acquired from a movable storage device such as a disk, USB storage, or from a database of an external system via the Internet, or a storage device of yet another type. For the sake of conciseness, in all the embodiments described below, the data 310 is assumed to be acquired from a database 30 of a server of a medical institution via the internet.

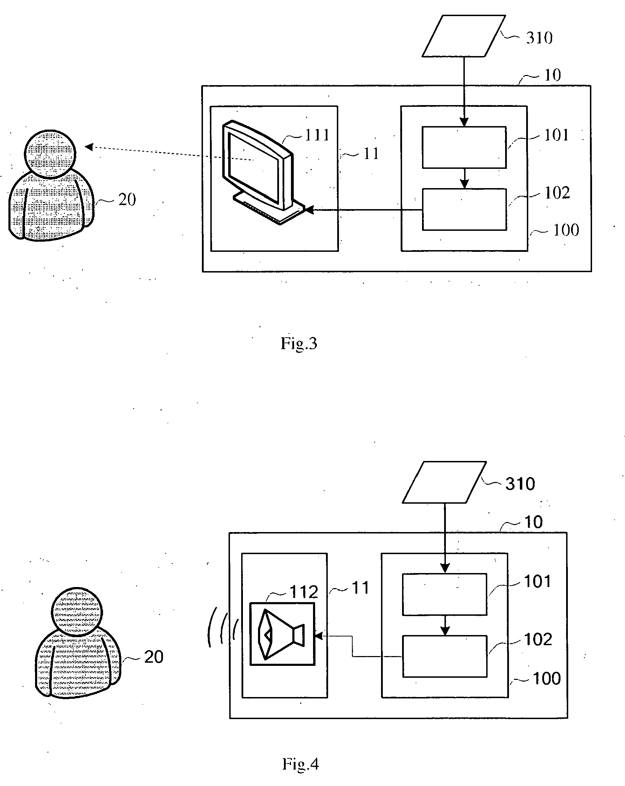

*15* Fig.3 illustrates a system according to an embodiment of the present invention. As shown in Fig.3, the system 10 comprises a first subsystem 11 and a controlling device 100 for controlling the system 11. The controlling device 100 comprises a first unit 101 for performing the above-mentioned step 1, and a second unit 102 for performing the above-mentioned step 2.

*16* The first subsystem 11 is used for providing audio/video content related to the set of exercises. Specifically, the first subsystem 11 comprises a first device 111, such as a display, for displaying video content associated with the set of exercises. This video content, aimed at guiding the user 20 to do the set of exercises for rehabilitation training, for example includes a text description describing tips and instructions about how to do the exercises; a demonstration picture or video showing how to do the series of exercises, and the like.

*17* First, in the system 10, the data 310 will be acquired by the first unit 101. The data 310 comprises information relating to a set of exercises to be done by the user 20. Besides, the data 310 also comprises information relating to characteristics of the user 20, especially the eyesight condition of the user 20.

*18* According to the eyesight condition of the user 20, the size of the content being displayed by the first device 111, i.e. the parameter of the first subsystem 11, will be adjusted by the second unit 102, which corresponds to step 2 of the above method. For example, if the user's 20 eyesight condition is normal, the second unit 102 will control the first device 111 to display video content in a normal size; if the user 20 has poor eyesight condition, the second unit 102 will control the first device 111 to display video content in a comparatively large size, so that the user 20 can see the content displayed on the screen more clearly.

*19* In a variation of this embodiment, the second unit 102 could adjust the distance between the first device 111 and the user 20 according to the eyesight condition of the user 20. For example, if the first device 111 is movable, the second unit 102 will control the device 111 so as to move it towards the user

20 to shorten the distance between the first device 111 and the user 20 when the user 20 has poor eyesight condition. Alternatively, the second unit 102 may instruct the user 20 to move towards the first device 111. Alternatively, the second unit 102 will adjust both the size of the content displayed by the first device 111 and the distance between the first device 111 and the user 20.

*20*. Fig.4 illustrates a system according to another embodiment of the present invention. In this example, similarly to the above embodiment, the system 10 comprises a controlling device 100 and a first subsystem 11. The first subsystem 11 is for providing audio and/or video content related to the set of exercises. Specifically, the first subsystem 11 comprises a second device 112, such as a loudspeaker, for playing audio content associated with the set of exercises. The user 20 could follow the audio content to do the set of exercises for rehabilitation training.

*21* According to this embodiment, the data 310 comprises information relating to a set of exercises to be done by the user 20. Besides, the data 310 also comprises information relating to characteristics of the user 20, especially the hearing condition of the user 20.

*22* According to the hearing condition of the user 20, the output volume of the content being played by the second device 112, i.e. the parameter of the first subsystem 11, will be adjusted by the second unit 102, which corresponds to step 2 of the above method. For example, if the user 20 has normal hearing condition, the second unit 102 will control the second device 112 to play audio content at normal volume; if the user 20 has poor hearing condition, the second unit 102 will control the second device 112 to play audio content at comparatively high volume, so that the user 20 can hear the audio content more clearly.

*23* In a variation of this embodiment, the second unit 102 could adjust the distance between the second device 112 and the user 20 according to the hearing condition of the user 20. For example, if the second device 112 is movable, the second unit 102 will control the second device 112 so as to move it towards the user 20 to shorten the distance between them when the user 20 has poor hearing condition. Alternatively, the second unit 102 may instruct the user 20 to move towards the second device 112. Alternatively, the second unit 102 can adjust both the output volume of the audio content played by the second device 112 and the distance between the second device 112 and the user 20.

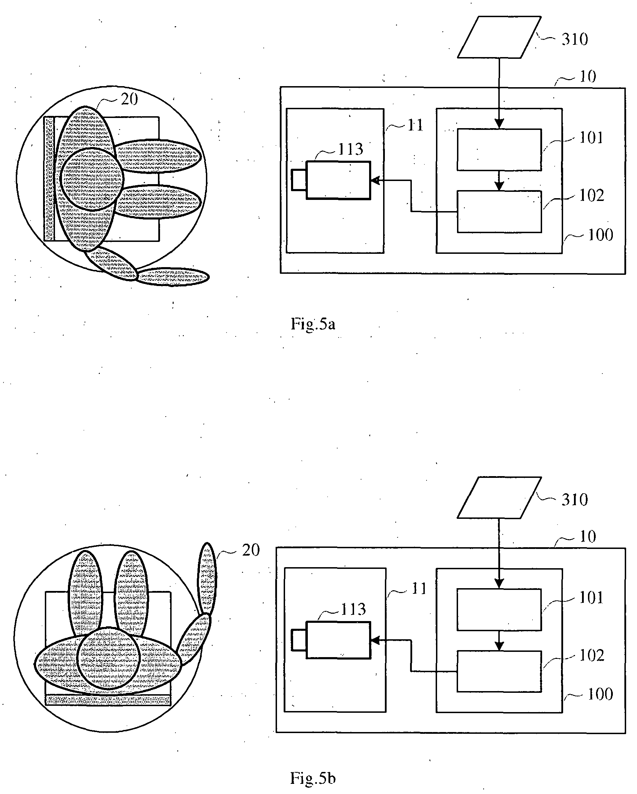

*24* Fig.5a and Fig.5b illustrate a system according to another embodiment of the present invention. Similarly to the above two embodiments, the system 10 comprises a controlling device 100 and a first subsystem 11.

*25* First, in the system 10 according to this embodiment, the data 310 will be acquired by the first unit 101, which corresponds to step 1 of the above method. The data 310 comprises information relating to a set of exercises to be done by the user 20.

*26* The first subsystem 11 is for providing audio/video content related to the set of exercises. Specifically, the first subsystem 11 comprises a camera 113 for generating video content associated with the set of exercises done by the user 20. More specifically, the camera 113 is an infrared camera. The user 20 could wear retroreflective markers at the joints of his trunk. The track of the retroreflective markers could be captured by the camera 113 and the movements of the user 20 could be recognized by an analyzing unit (not shown in the Figures) of the system 10. The movements of the user 20 might be compared with the set of exercises to be done by the user 20 by the same analyzing unit as that used for recognizing the motion of the user 20 or by another, separate analyzing unit (not shown in the Figures) of the system 10. Subsequently, instructions might be given, for example via the display 111 or the loudspeaker 112, to the user 20 according to the comparison result, so that the user 20 can correct his movements accordingly. Although different retroreflective markers may be mislabeled when they are close to one another, demonstrate an overlap, or are occluded, good monitoring images or video images can still be obtained by movement of the camera 113.

*27* The camera 113 could be mounted to one end of a robot arm controlled by the second unit 102. The second unit 102 could adjust at least one of location and angle of the camera 113 according to the set of exercises to be done by the user 20, so as to move the camera 113 to find a better view of the user 20. For example, if the set of exercises to be done by the user 20 mainly consists of arm movements in a plane parallel to the body, the camera 113 could be moved to the front side of the user 20, as illustrated in Fig.5a; if the set of exercises to be done by the user 20 mainly consists of right arm motions in a plane vertical to the body, the camera 113 could be moved to the right side of the user 20, as illustrated in Fig.5b. It will be understood by those skilled in the art that the cases illustrated in Fig.5a and Fig.5b are exemplary rather than restricted to this embodiment. The camera 113 could also be moved to the left side of the user 20, above the top side of the user 20, or to yet another location with respect to the user 20.

*28* In a variation of this embodiment, the camera 113 is a common camera sensing visible light. Pictures or video of the user 20 are captured by the camera 113 and the movements of the user 20 could be recognized by the system 10 by virtue of image recognition technology. It will be understood by those skilled in the art that any existing image recognition technology could be used in this embodiment, however, further details thereof will not be given herein.

*29* In another variation of this embodiment, the data 310 comprises information relating to a set of exercises to be done by the user 20 as well as information relating to characteristics of the user 20. The second unit 102 could adjust a parameter of the first subsystem 11 according to the data 310. For example, if the set of exercises to be done by the user 20 mainly consists of arm movements and the characteristics comprise the height of the user 20, the second unit 102 could adjust the location and/or the angle of the camera 113 according to the arm movements and the height of the user 20, e.g. the camera 113 might be moved so as to be horizontally level with the shoulders of the user 20.

*30* Of course, the system 10 comprising a plurality of cameras is feasible. However, it will be obvious that the system 10 with a single camera 113 is more cost-efficient.

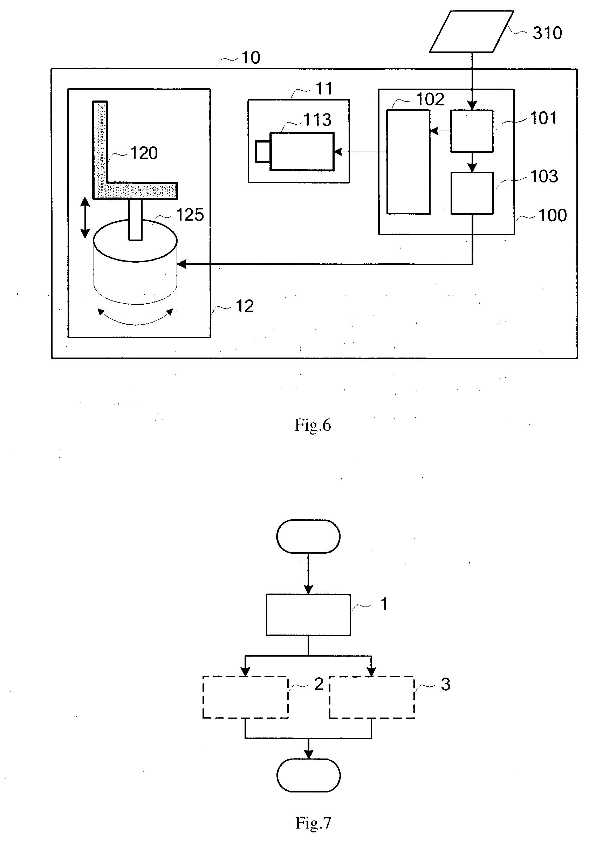

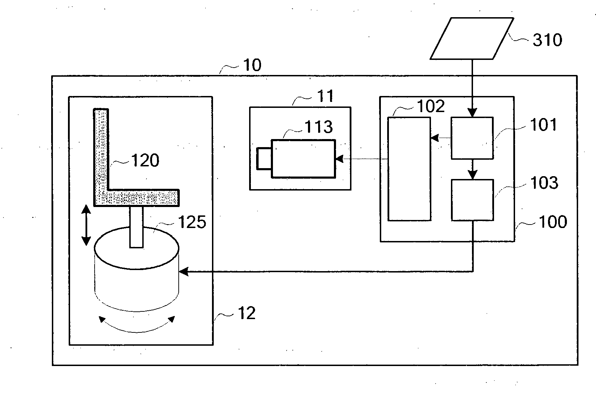

*31* Fig.6 illustrates a system according to another embodiment of the present invention. Similarly to the aforementioned embodiments, the system 10 comprises a controlling device 100 and a first subsystem 11. In addition, the system 10 further comprises a second subsystem 12.

*32* The second subsystem 12 serves for supporting the user's body when the user 20 is doing the set of exercises. It will be understood by those skilled in the art that the second subsystem 12 could be embodied as a plurality of structures, such as a plate, a bed, a chair, straps for resisting movement of the user 20, or yet another structure.

*33* Fig.7 illustrates a flowchart for controlling the system 10 as shown in Fig.6. As shown in Fig.7, the method comprises three steps. Step 1 is for acquiring previously generated data 310 which is related to the user 20. Step 2 is performed, subsequent to step 1, for adjusting a parameter of the first subsystem 11 according to the data 310. Step 3 is performed, subsequent to step 1, for adjusting a parameter of the second subsystem 12 according to the data 310.

*34* It should be understood that step 2 and step 3 are optional. More specifically, in some cases, step 1 and step 2 are performed; in some other cases, step 1 and step 3 are performed, whereas in yet other cases, all of the steps 1, 2 and 3 are performed.

*35* Specifically, in the system 10 as shown in Fig.6, the second system 12 comprises a chair 120 and a motor 125 mounted underneath the chair 120. The motor 125 could be controlled so as to adjust the height of the chair 120 or rotate the chair 120.

*36* The controlling device 100 comprises a first unit 101, a second unit 102 and a third unit 103. The first unit 101 is for performing the abovementioned step 1, the second unit 102 is for performing the abovementioned step 2, and the third unit 103 is for performing the abovementioned step 3.

*37* In this embodiment, the data 310 comprises information relating to a set of exercises which is intended to be done by the user 20. The camera 113, which might be fixed, generates video content of the set of exercise done by the user 20, and the motions of the user 20 could be recognized by the system 10. The system 10 might compare the motions of the user 20 with the set of exercises intended to be done by the user 20. And then, instructions might be given, for example via a display or a loudspeaker, to the user 20 according to the compare result, so that the user 20 could correct his motions accordingly.

*38* According to the set of user 20 exercises in the data 310, the third unit 103 could adjust at least one of height and angle of the chair 120 by controlling the motor 125 so as to move the chair 120 to find a better position of the user 20 with respect to the camera 113. For example, if the set of exercises to be done by the user 20 mainly consists of arm movements in a plane parallel to the body, the chair 120 could be adjusted so that the user 20 directly faces the camera 113; if the set of exercises to be done by the user 20 mainly consists of arm movements in a plane vertical to the body, the chair 120 could be adjusted so that the camera 113 is on the right or left side of the user 20. It will be understood by those skilled in the art that the chair 120 could also be adjusted underneath the camera 113, or yet another location with respect to the camera 113.

*39* In a variation of this embodiment, the camera 113 is movable. According to the set of exercises to be done by the user 20, the second unit 102 will adjust at least one of location and angle of the camera 113; simultaneously, the third unit 103 will adjust at least one of height and angle of the chair 120 by controlling the motor 125 so as to find a better position of the user 20 with respect to the camera 113.

*40* In another variation of this embodiment, the chair 120 might be fixed and the data 310 comprises information relating to characteristics of the user 20, such as the height of the user 20. After the data 310 is acquired by the first unit 101, the second unit 102 could at least adjust the location of the camera 113 according to the height of the user 20.

*41* Yet in another variation of this embodiment, the data 310 comprises information relating to the set of exercises to be done by the user 20 as well as information relating to characteristics of the user 20, such as the height of the user 20. For example, the first subsystem 11 might comprise a first device 111 for displaying video content associated with the set of exercises and/or a second device 112 for playing audio content associated with the set of exercises. After the data 310 is acquired by the first unit 101, the second unit 102 will adjust a parameter of the first subsystem 11; simultaneously, the third unit 103 will adjust a parameter of the second subsystem 12. The parameter of the first subsystem 11 adjusted by the second unit 102 might correspond to the size of video content displayed by the first device 111 and/or the output volume of audio content played by the second device 112. The parameter of the second subsystem 12 adjusted by the third unit 103 might correspond to at least one of height and angle of the chair 120.

*42* The controlling device 100 of the invention could be either integrated within the system 10 or be separate from the system 10, which means that the device 100 could be sold/purchased separately. The units in the device 100 could be implemented by software, hardware or a combination of both. For example, the function unit could be implemented by a microprocessor with instruction code stored in a memory. In yet another example, the function unit could be implemented by a chip.

*43* To summarize the invention, data relating to characteristics of the user could be used to adjust only one or more parameters of the first subsystem or only one or more parameters of the second subsystem, or to adjust different parameters of both subsystems; similarly, data relating to said set of exercises could also be used to adjust only one or more parameters of the first subsystem or only the second subsystem or to adjust both subsystems; it is also possible to use data comprising information relating to characteristics of the user as well as to said set of exercises to adjust only the first subsystem or only the second subsystem or to adjust both subsystems.

*44* It will be understood by those skilled in the art that the disclosed embodiments are exemplary only and are not restrictive to the present invention. Different features disclosed in different embodiments could be combined for the purpose of achieving advantages.

*45* Other variations to the disclosed embodiments can be understood and effected by those skilled in the art in practicing the claimed invention, from a study of the drawings, the disclosure and the appended claims. In the claims, the term "comprise/comprising" does not exclude other means or steps; the indefinite article "a" or "an" does not exclude a plurality; the terms "first", "second" are used for distinguishing between elements and do not denote any specific order. Any reference signs in the claims shall not be construed as limiting the scope. The functions of several items recited in the claims may be fulfilled by a single hardware or software module. The mere fact that certain measures are recited in mutually different dependent claims does not indicate that a combination of these measures cannot be used to advantage.

## Claims

1. A method of controlling a system (10) for providing support to a user (20) to do a set of exercises, said system comprising a first subsystem (11) for providing audio and/or video content related to the set of exercises to the user (20), said method comprising the steps of:
- acquiring previously generated data (310) comprising information relating to said set of exercises; and
- adjusting a parameter of said first subsystem (11) according to said data (310),
the method being **characterized in that**
said parameter of the first subsystem corresponds to at least one of location and shooting angle of a camera (113) according to the data (310), said first subsystem (11) comprising the camera (113) configured to acquire and generate video content associated with the exercises done by the user (20).

2. A method according to claim 1, wherein said system (10) further comprises a second subsystem (12) for supporting the user's body when the user (20) does said set of exercises, said method further comprising a step of adjusting a parameter of said second subsystem (12) according to said data (310).

3. A method according to claim 1 or 2, wherein said information relating to characteristics of said user corresponds to the eyesight condition of said user (20), said first subsystem (11) further comprising a display (111) for displaying video content associated with said set of exercises; said parameter of the first subsystem corresponding to the size of the content to be displayed.

4. A method according to claim 1 or 2, wherein said information relating to characteristics of said user corresponds to the hearing condition of said user (20), the first subsystem (11) further comprising a speaker (112) configured for playing audio content related to said set of exercises; said parameter of the first subsystem corresponds to the output volume of the audio content to be played.

5. A method according to claim 2, wherein the second subsystem (12) comprises a chair (120); said parameter of the second subsystem corresponds to at least one of height and angle of the chair.

6. A system (10) for providing support to a user (20) to do a set of exercises, the system comprising
- a first subsystem (11) for providing audio and/or video content related to the set of exercises to the user (20),:
- a first unit (101) configured to acquire previously generated data (310) comprising information relating to said set of exercises;
- a second unit (102) configured to adjust said first subsystem (11) according to said data (310), wherein the first subsystem (11) comprises a camera (113) configured to acquire and generate video content associated with the set of exercises done by the user, and the second unit (102) is further configured to adjust at least one of location and shooting angle of the camera (113).

7. A system according to claim 6, wherein said system (10) further comprises a second subsystem (12) for supporting the user's body when the user (20) is doing said set of exercises, and said system further comprising a third unit configured to adjust a parameter of said second subsystem (12) according to said data (310).

8. A system according to claim 6 or 7, wherein the first subsystem (11) further comprises a display (111) configured to provide video content associated with the set of exercises to the user (20), the data (310) comprises information relating to eyesight condition of said user (20), and the second unit (102) is further configured to adjust the size of the content to be displayed.

9. A system according to claim 6 or 7, wherein said system (10) further comprises a speaker (112) configured to provide audio content associated with the set of exercises to the user (20), the data (310) comprises information relating to hearing condition of said user (20), and the second unit (102) is further configured to adjust the output volume of the speaker (112).

10. A system according to claim 7, wherein said second subsystem (12) comprises a chair (120); said parameter of the second subsystem corresponds to at least one of height and angle of the chair.

## Patentansprüche

1. Verfahren, um ein System (10) so zu steuern, dass ein Benutzer (20) bei der Ausführung eines Übungssatzes unterstützt wird, wobei das System ein erstes Subsystem (11) umfasst, um für den Benutzer (20) einen auf den Übungssatz bezogenen Audio-und/oder Videoinhalt bereitzustellen, wobei das Verfahren die folgenden Schritte umfasst, wonach:
- zuvor erzeugte Daten (310) mit auf den Übungssatz bezogenen Informationen erfasst werden; und
- ein Parameter des ersten Subsystems (11) den Daten (310) entsprechend eingestellt wird,
wobei das Verfahren **dadurch gekennzeichnet ist, dass**
der Parameter des ersten Subsystems zumindest der Position oder dem Aufnahmewinkel einer Camera (113) gemäß den Daten (310) entspricht, wobei das erste Subsystem (11) die Camera (113) umfasst, die so eingerichtet ist, dass sie den Videoinhalt erfasst und erzeugt, der den durch den Benutzer (20) ausgeführten Übungen zugeordnet ist.

2. Verfahren nach Anspruch 1, wobei das System (10) weiterhin ein zweites Subsystem (12) umfasst, um den Körper des Benutzers zu unterstützen, wenn der Benutzer (20) den Übungssatz ausführt, wobei das Verfahren weiterhin einen Schritt des Einstellens eines Parameters des zweiten Subsystems (12) den Daten (310) entsprechend umfasst.

3. Verfahren nach Anspruch 1 oder 2, wobei die auf die charakteristischen Merkmale des Benutzers bezogenen Informationen dem Zustand des Sehvermögens des Benutzers (20) entsprechen, wobei das erste Subsystem (11) weiterhin ein Display (111) umfasst, um den dem Übungssatz zugeordneten Videoinhalt darzustellen, wobei der Parameter des ersten Subsystems der Größe des darzustellenden Inhalts entspricht.

4. Verfahren nach Anspruch 1 oder 2, wobei die auf die charakteristischen Merkmale des Benutzers bezogenen Informationen dem Zustand des Hörvermögens des Benutzers (20) entsprechen, wobei das erste Subsystem (11) weiterhin einen Lautsprecher (112) umfasst, der so eingerichtet ist, dass er den auf den Übungssatz bezogenen Audioinhalt wiedergibt, wobei der Parameter des ersten Subsystems dem Ausgangsvolumen des wiederzugebenden Audioinhalts entspricht.

5. Verfahren nach Anspruch 2, wobei das zweite Subsystem (12) einen Stuhl (120) umfasst, wobei der Parameter des zweiten Subsystems zumindest der Höhe oder dem Winkel des Stuhls entspricht.

6. System (10), um einen Benutzer (20) bei der Ausführung eines Übungssatzes zu unterstützen, wobei das System umfasst:
- ein erstes Subsystem (11), um für den Benutzer (20) einen auf den Übungssatz bezogenen Audio- und/oder Videoinhalt bereitzustellen;
- eine erste Einheit (101), die so eingerichtet ist, dass sie zuvor erzeugte Daten (310) mit auf den Übungssatz bezogenen Informationen erfasst;
- eine zweite Einheit (102), die so eingerichtet ist, dass sie das erste Subsystem (11) den Daten (310) entsprechend einstellt, wobei das erste Subsystem (11) eine Camera (113) umfasst, die so eingerichtet ist, dass sie den Video inhalt erfasst und erzeugt, der den durch den Benutzer ausgeführten Übungen zugeordnet ist, und wobei die zweite Einheit (102) weiterhin so eingerichtet ist, dass sie zumindest die Position oder den Aufnahmewinkel der Camera (113) einstellt.

7. System nach Anspruch 6, wobei das System (10) weiterhin ein zweites Subsystem (12) umfasst, um den Körper des Benutzers zu unterstützen, wenn der Benutzer (20) den Übungssatz ausführt, und wobei das System weiterhin eine dritte Einheit umfasst, die so eingerichtet ist, dass sie einen Parameter des zweiten Subsystems (12) den Daten (310) entsprechend einstellt.

8. System nach Anspruch 6 oder 7, wobei das erste Subsystem (11) weiterhin ein Display (111) umfasst, das so eingerichtet ist, dass es den dem Übungssatz zugeordneten Videoinhalt für den Benutzer (20) bereitstellt, wobei die Daten (310) auf den Zustand des Sehvermögens des Benutzers (20) bezogene Informationen umfassen, und wobei die zweite Einheit (102) weiterhin so eingerichtet ist, dass sie die Größe des darzustellenden Inhalts einstellt.

9. System nach Anspruch 6 oder 7, wobei das System (10) weiterhin einen Lautsprecher (112) umfasst, der so eingerichtet ist, dass er dem Benutzer (20) den auf den Übungssatz bezogenen Audioinhalt wiedergibt, wobei die Daten (310) auf den Zustand des Hörvermögens des Benutzers (20) bezogene Informationen umfassen, und wobei die zweite Einheit (102) weiterhin so eingerichtet ist, dass sie das Ausgangsvolumen des Lautsprechers (112) einstellt.

10. System nach Anspruch 7, wobei das zweite Subsystem (12) einen Stuhl (120) umfasst, wobei der Parameter des zweiten Subsystems zumindest der Höhe oder dem Winkel des Stuhls entspricht.

## Revendications

1. Procédé de commande d'un système (10) de fourniture de soutien à un utilisateur (20) pour qu'il fasse un ensemble d'exercices, ledit système comprenant un premier sous-système (11) de fourniture de contenu audio et/ou vidéo lié à l'ensemble d'exercices à l'utilisateur (20), ledit procédé comprenant les étapes :
- d'acquisition de données préalablement générées (310) comprenant des informations relatives audit ensemble d'exercices ; et
- de réglage d'un paramètre dudit premier sous-système (11) selon lesdites données (310),
le procédé étant **caractérisé en ce que**
ledit paramètre du premier sous-système correspond à au moins l'un d'un emplacement et d'un angle de prise de vue d'une caméra (113) selon les données (310), ledit premier sous-système (11) comprenant la caméra (113) configurée pour acquérir et générer un contenu vidéo associé aux exercices faits par l'utilisateur (20).

2. Procédé selon la revendication 1, dans lequel ledit système (10) comprend en outre un second sous-système (12) de soutien du corps de l'utilisateur lorsque l'utilisateur (20) fait ledit ensemble d'exercices, ledit procédé comprenant en outre une étape de réglage d'un paramètre dudit second sous-système (12) selon lesdites données (310).

3. Procédé selon la revendication 1 ou 2, dans lequel lesdites informations relatives à des caractéristiques dudit utilisateur correspondent à l'état de la vision dudit utilisateur (20), ledit premier sous-système (11) comprenant en outre un afficheur (111) pour afficher un contenu vidéo associé audit ensemble d'exercices ; ledit paramètre du premier sous-système correspondant à la taille du contenu à afficher.

4. Procédé selon la revendication 1 ou 2, dans lequel lesdites informations relatives à des caractéristiques dudit utilisateur correspondent à l'état de l'audition dudit utilisateur (20), le premier sous-système (11) comprenant en outre un haut-parleur (112) configuré pour lire un contenu audio lié audit ensemble d'exercices ; ledit paramètre du premier sous-système correspond au volume de sortie du contenu audio à lire.

5. Procédé selon la revendication 2, dans lequel le second sous-système (12) comprend une chaise (120) ; ledit paramètre du second sous-système correspond à au moins l'un d'une hauteur et d'un angle de la chaise.

6. Système (10) de fourniture de soutien à un utilisateur (20) pour qu'il fasse un ensemble d'exercices, le système comprenant
- un premier sous-système (11) de fourniture de contenu audio et/ou vidéo lié à l'ensemble d'exercices à l'utilisateur (20) ;
- une première unité (101) configurée pour acquérir des données préalablement générées (310) comprenant des informations relatives audit ensemble d'exercices ;
- une deuxième unité (102) configurée pour régler ledit premier sous-système (11) selon lesdites données (310),
dans lequel le premier sous-système (11) comprend une caméra (113) configurée pour acquérir et générer un contenu vidéo associé à l'ensemble d'exercices faits par l'utilisateur, et la deuxième unité (102) est en outre configurée pour régler au moins l'un d'un emplacement et d'un angle de prise de vue de la caméra (113).

7. Système selon la revendication 6, dans lequel ledit système (10) comprend en outre un second sous-système (12) de soutien du corps de l'utilisateur lorsque l'utilisateur (20) fait ledit ensemble d'exercices, et ledit système comprenant en outre une troisième unité configurée pour régler un paramètre dudit second sous-système (12) selon lesdites données (310).

8. Système selon la revendication 6 ou 7, dans lequel le premier sous-système (11) comprend en outre un afficheur (111) configuré pour fournir un contenu vidéo associé à l'ensemble d'exercices à l'utilisateur (20), les données (310) comprennent des informations relatives à l'état de la vision dudit utilisateur (20), et la deuxième unité (102) est en outre configurée pour régler la taille du contenu à afficher.

9. Système selon la revendication 6 ou 7, dans lequel ledit système (10) comprend en outre un haut-parleur (112) configuré pour fournir un contenu audio associé à l'ensemble d'exercices à l'utilisateur (20), les données (310) comprennent des informations relatives à l'état de l'audition dudit utilisateur (20), et la deuxième unité (102) est en outre configurée pour régler le volume de sortie du haut-parleur (112).

10. Système selon la revendication 7, dans lequel ledit second sous-système (12) comprend une chaise (120) ; ledit paramètre du second sous-système correspond à au moins l'un de la hauteur et de l'angle de la chaise.
